(19) **Europäisches Patentamt**

**European Patent Office**

**Office européen des brevets**

(11) **EP 1 078 604 B1**

(12) **EUROPEAN PATENT SPECIFICATION**

(45) Date of publication and mention
of the grant of the patent:
**20.10.2004  Bulletin 2004/43**

(51) Int Cl.[7]: **A61B 18/18**, A61N 5/06

(21) Application number: **00124370.8**

(22) Date of filing: **05.04.1993**

(54) **Device for therapeutic electromagnetic treatment**

Gerät zur elektromagnetischen Therapie

Appareil pour la thérapie électromagnétique

(84) Designated Contracting States:
**AT BE CH DE DK ES FR GB GR IE IT LI LU MC NL
PT SE**

(30) Priority: **09.04.1992  IL  10154792
20.10.1992  US 964210**

(43) Date of publication of application:
**28.02.2001  Bulletin 2001/09**

(62) Document number(s) of the earlier application(s) in
accordance with Art. 76 EPC:
**93302662.7 / 0 565 331**

(73) Proprietor: **ESC Medical Systems Ltd.
Haifa (IL)**

(72) Inventor: **Eckhouse, Shimon
Haifa 34987 (IL)**

(74) Representative: **Altenburg, Udo, Dipl.-Phys. et al
Patent- und Rechtsanwälte
Bardehle . Pagenberg . Dost .
Altenburg - Geissler
Galileiplatz 1
81679 München (DE)**

(56) References cited:
**WO-A-91/15264          US-A- 3 693 623
US-A- 4 608 978          US-A- 4 792 341
US-A- 4 860 172          US-A- 4 940 922**

EP 1 078 604 B1

## Description

The present invention relates generally to the art of therapeutic electromagnetic treatment and more specifically to an apparatus for utilising a spatially extended pulsed light source such as a flashlamp (flash tube) for such a treatment.

It is known in the prior art to use electromagnetic radiation in medical application for therapeutic uses such as treatment of skin disorders. For example, US-A-4,298,005 (Mutzhas) describes a continuous ultraviolet lamp with cosmetic, photobiological, and photochemical applications. A treatment based on using the UV portion of the spectrum and its photochemical interaction with the skin is described. The power delivered to the skin using Mutzhas' lamp is described as 150W/m$^2$, which does not have a significant effect on skin temperature.

In addition to prior art treatment involving UV light, lasers have been used for dermatological procedures, including Argon lasers, $CO_2$ lasers, Nd(Yag) lasers, Copper vapor lasers, ruby lasers and dye lasers. For example, US-A-4,829,262 (Furumoto), describes a method of constructing a dye laser used in dermatology applications. Two skin conditions which may be treated by laser radiation are external skin irregularities such as local differences in the pigmentation or structure of the skin, and vascular disorders lying deeper under the skin which cause a variety of skin abnormalities including port wine stains, telangiectasias, leg veins and cherry and spider angiomas. Laser treatment of these skin disorders generally includes localised heating of the treatment area by absorption of laser radiation. Heating the skin changes or corrects the skin disorder and causes the full or partial disappearance of the skin abnormality.

Certain external disorders such as pigmented lesions can also be treated by heating the skin very fast to a high enough temperature to evaporate parts of the skin. Deeper-lying vascular disorders are more typically treated by heating the blood to a high enough temperature to cause it to coagulate. The disorder will then eventually disappear. To control the treatment depth a pulsed radiation source is often used. The depth the heat penetrates in the blood vessel is controlled by controlling the pulse width of the radiation source. The absorption and scattering coefficients of the skin also affect the heat penetration. These coefficients are a function of the constituents of skin and the wavelength of the radiation. Specifically, the absorption coefficient of light in the epidermis and dermis tends to be a slowly varying, monotonically decreasing function of wavelength. Thus, the wavelength of the light should be chosen so that the absorption coefficient is optimised for the particular skin condition and vessel size being treated.

The effectiveness of lasers for applications such as tattoo removal and removal of birth and age marks is diminished because lasers are monochromatic. A laser of a given wavelength may be effectively used to treat a first type of skin pigmentation disorder, but, if the specific wavelength of the laser is not absorbed efficiently by skin having a second type of disorder, it will be ineffective for the second type of skin disorder. Also, lasers are usually complicated, expensive to manufacture, large for the amount of power delivered, unreliable and difficult to maintain.

The wavelength of the light also affects vascular disorder treatment because blood content in the vicinity of the vascular disorders varies, and blood content affects the absorption coefficient of the treatment area. Oxyhemoglobin is the main chromophore which controls the optical properties of blood and has strong absorption bands in the visible region. More particularly, the strongest absorption peak of oxyhemoglobin occurs at 418nm and has a band-width of 60nm. Two additional absorption peaks with lower absorption coefficients occur at 542 and 577nm. The total band-width of these two peaks is on the order of 100nm. Additionally, light in the wavelength range of 500 to 600nm is desirable for the treatment of blood vessel disorders of the skin since it is absorbed by the blood and penetrates through the skin. Longer wavelengths up to 1000nm are also effective since they can penetrate deeper into the skin, heat the surrounding tissue and, if the pulse-width is long enough, contribute to heating the blood vessel by thermal conductivity. Also, longer wavelengths are effective for treatment of larger diameter vessels because the lower absorption coefficient is compensated for by the longer path of light in the vessel.

In addition to being used for treating skin disorders, lasers have been used for invasive medical procedures such as lithotripsy and removal of blood vessel blockage. In such invasive procedures laser light is coupled to optical fibres and delivered through the fibre to the treatment area. In lithotripsy the fibre delivers light from a pulsed laser to a kidney or gallstone and the light interaction with the stone creates a shock wave which pulverises the stone. To remove blood vessel blockage the light is coupled to the blockage by the fibre and disintegrates the blockage. In either case the shortcomings of lasers discussed above with respect to laser skin treatment are present. Accordingly, a treatment device for lithotripsy and blockage removal utilising a flashlamp would be desirable.

To effectively treat an area the light from the source must be focused on the treatment area. Coupling pulsed laser light into optical fibres in medicine is quite common. The prior art describes coupling isotropic incoherent point sources such as CW lamps into small optical fibres. For example, US-A-4,757,431 (Cross, et al.) discloses a method for focusing incoherent point sources with small filaments or an arc lamp with an electrode separation of 2mm into a small area. Point (or small) sources are relatively easy to focus without large losses in energy because of the small size of the source. Also, US-A-4,022,534 (Kishner) discloses light produced by a flash tube and the collection of only a small portion of the light emitted by the tube into an optical fibre.

However, the large dimension of an extended source such as a flashlamp make it difficult to focus large fractions of its energy into small areas. Coupling into optical fibres is even more difficult since not only must a high energy density be achieved, but the angular distribution of the light has to be such that trapping in the optical fibre can be accomplished.

From US-A-4 940 922 a flashlamp operable to provide a pulsed light output is known. The pulses range from about two to ten microseconds.

In order to solve the technical problems outlined above including the specifity of prior art systems and their technical complexity and expense, the device of the present invention is defined by the features of claim 1. Advantageous embodiments of the invention are claimed in claims 2 to 6.

A wide band electromagnetic radiation source that covers the near UV and the visible portion of the spectrum would be desirable for treatment of external skin and vascular disorders. The overall range of wavelengths of the light source should be sufficient to optimise treatment for any of a number of applications. Such a therapeutic electromagnetic radiation device should also be capable of providing an optimal wavelength range within the overall range for the specific disorder being treated. The intensity of the light should be sufficient to cause the required thermal effect by raising the temperature of the treatment area to the required temperature. Also, the pulse-width should be variable over a wide enough range so as to achieve the optimal penetration depth for each application. Therefore, it is desirable to provide a light source having a wide range of wavelengths, which can be selected according to the required skin treatment, with a controlled pulse-width and a high enough energy density for application to the affected area.

Pulsed non-laser type light sources such as linear flashlamps provide these benefits. The intensity of the emitted light can be made high enough to achieve the required thermal effects. The pulse-width can be varied over a wide range so that control of thermal depth penetration can be accomplished. The typical spectrum covers the visible and ultraviolet range and the optical bands most effective for specific applications can be selected, or enhanced using fluorescent materials. Moreover, non-laser type light sources such as flashlamps are much simpler and easier to manufacture than lasers, are significantly less expensive for the same output power and have the potential of being more efficient and more reliable. They have a wide spectral range that can be optimised for a variety of specific skin treatment applications. These sources also have a pulse length that can be varied over a wide range which is critical for the different types of skin treatments.

For a better understanding of the invention, reference is made to the accompanying diagrammatic drawings, in which:

Figure 1 is a cross-sectional view of an incoherent, pulsed light source skin treatment device;

Figure 2 is a side view of the light source of Figure 1;

Figure 3 is a schematic diagram of a pulse forming network with a variable pulse width for use with the skin treatment device of Figures 1 and 2.

In the various figures, like reference numerals are used to describe like components.

Before explaining at least one embodiment of the invention in detail it is to be understood that the invention is not limited in its application to the details of construction and the arrangement of the components set forth in the following description or illustrated in the drawings. The invention is capable of other embodiments or of being practised or carried out in various ways. Also, it is to be understood that the phraseology and terminology employed herein is for the purpose of description and should not be regarded as limiting.

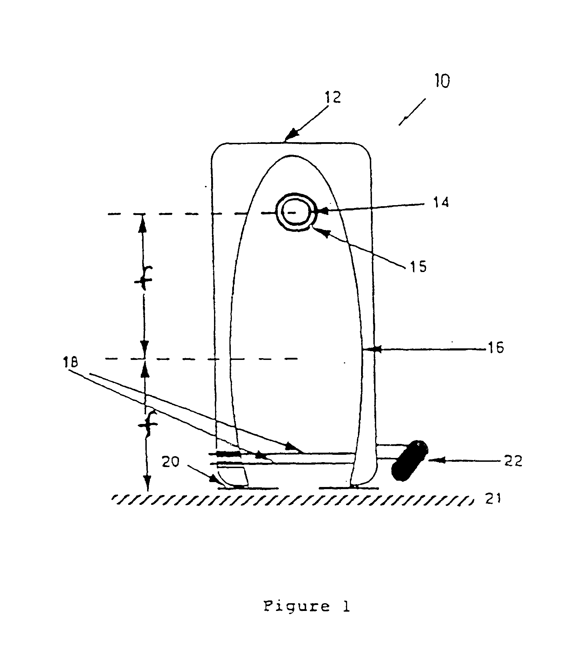

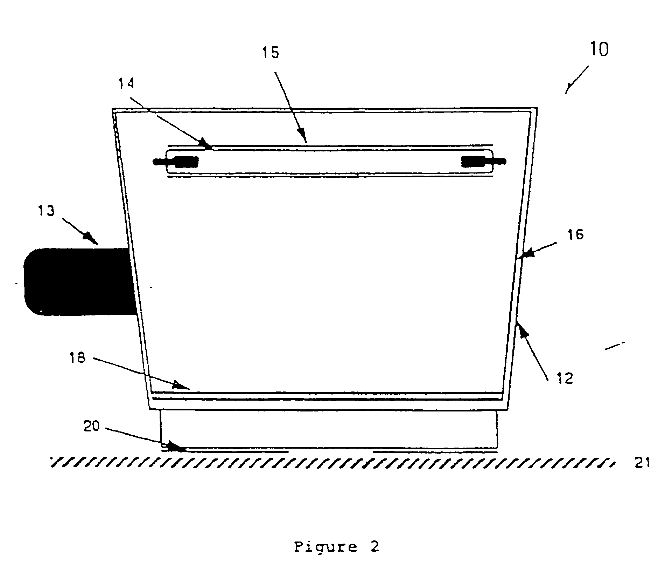

Referring now to Figures 1 and 2, cross-sectional and side views of an incoherent, pulsed light source skin treatment device 10 constructed and operated in accordance with the principles of the present invention are shown. The device 10 may be seen to include a housing 12, having an opening therein, a handle 13 (Figure 2 only), a light source 14 having an outer glass tube 15, an elliptical reflector 16, a set of optical filters 18, an iris 20 and a detector 22 (Figure 1 only). Light source 14, which is mounted in housing 12, may be a typical incoherent light source such as a gas filled linear flashlamp Model No. L5568 available from ILC. The spectrum of light emitted by gas filled linear flashlamp 14 depends on current density, type of glass envelope material and gas mixture used in the tube. For large current densities (e.g., 3000 A/cm$^2$ or more) the spectrum is similar to a black body radiation spectrum. Typically, most of the energy is emitted in the 300 to 1000nm wavelength range.

To treat a skin (or visible) disorder a required light density on the skin must be delivered. This light density can be achieved with the focusing arrangement shown in Figures 1 and 2. Figure 1 shows a cross-section view of reflector 16, also mounted in housing 12. As shown in Figure 1, the cross-section of reflector 16 in a plane is perpendicular to the axis of flashlamp 14 is an ellipse. Linear flashlamp 14 is located at one focus of the ellipse and reflector 16 is positioned in such a way that the treatment area of skin 21 is located at the other focus. The arrangement shown is similar to focusing arrangements used with lasers and efficiently couples light from flashlamp 14 to the skin. This arrangement should not, however, be considered limiting. Elliptical reflector 16 may be a metallic reflector, typically polished aluminum which is an easily machinable reflector and has a very high reflectivity in the visible, and the UV range of the spectrum can be used. Other bare or coated metals can also be used for this purpose.

Optical and neutral density filters 18 are mounted in housing 12 near the treatment area and may be moved into the beam or out of the beam to control the spectrum and intensity of the light. Typically, 50 to 100nm band-width filters, as well as low cut-off filters in the visible and ultraviolet portions of the spectrum, are used. In some procedures it is desirable to use most of the spectrum, with only the UV portion being cut off. In other applications, mainly for deeper penetration, it is preferable to use narrower band-widths. The band-width filters and the cut-off filters are readily available commercially.

Glass tube 15 is located coaxially with flashlamp 14 and has fluorescent material deposited on it. Glass tube 15 will typically be used for treatment of coagulation of blood vessels to optimise the energy efficiency of device 10. The fluorescent material can be chosen to absorb the UV portion of the spectrum of flashlamp 14 and generate light in the 500 to 650nm range that is optimised for absorption in the blood. Similar materials are coated on the inner walls of commercial fluorescent lamps. A typical material used to generate "warm" white light in fluorescent lamps has a conversion efficiency of 80%, has a peak emission wavelength of 570nm and has a bandwidth of 70nm and is useful for absorption in blood. The few millisecond decay time of these phosphors is consistent with long pulses that are required for the treatment of blood vessels.

Other shapes or configurations of flashlamp 14 such as circular, helical, short arc and multiple linear flashlamps may be used. Reflector 16 may have other designs such as parabolic or circular reflectors. The light source can also be used without a reflector and the required energy and power density may be achieved by locating light source 14 in close proximity to the treatment area.

Iris 20 is mounted in housing 12 between optical filters 18 and the treatment area and controls the length and the width of the exposed area, i.e. by collimating the output of flashlamp 14. The length of flashlamp 14 controls the maximum length that can be exposed. Typically a 8cm long (arc length) tube will be used and only the central 5cm of the tube is exposed. Using the central 5cm assures a high degree of uniformity of energy density in the exposed skin area. Thus, in this embodiment the iris 20 (also called a collimator) will enable exposure of skin areas of a maximum length of 5cm. The iris 20 may be closed to provide a minimum exposure length of one millimetre. Similarly, the width of the exposed skin area can be controlled in the range of 1 to 5mm for a 5mm wide flashlamp. Larger exposed areas can be easily achieved by using longer flash tubes or multiple tubes, and smaller exposure areas are obtainable with an iris that more completely collimates the beam. The present invention provides a larger exposure area compared to prior art lasers or point sources and is very effective in the coagulation of blood vessels since blood flow interruption over a longer section of the vessel is more effective in coagulating it. The larger area exposed simultaneously also reduces the required procedure time.

Detector 22 (Figure 1) is mounted outside housing 12 and monitors the light reflected from the skin. Detector 22 combined with optical filters 18 and neutral density filters is used to achieve a quick estimate of the spectral reflection and absorption coefficients of the skin. This may be carried out at a low energy density level prior to the application of the main treatment pulse. Measurement of the optical properties of the skin prior to the application of the main pulse is useful to determine optimal treatment conditions. As stated above, the wide spectrum of the light emitted from the non-laser type source enables investigation of the skin over a wide spectral range and choice of optimal treatment wavelengths.

Detector 22 or a second detector system may also be used for real-time temperature measurement of the skin during its exposure to the pulsed light source. This is useful for skin thermolysis applications with long pulses in which light is absorbed in the epidermis and dermis. when the external portion of the epidermis reaches too high a temperature, permanent scarring of the skin may result. Thus, the temperature of the skin should be measured. This can be realised using infra-red emission of the heated skin, to prevent over-exposure.

A typical real-time detector system would measure the infra-red emission of the skin at two specific wavelengths by using two detectors and filters. The ratio between the signals of the two detectors can be used to estimate the instantaneous skin temperature. The operation of the pulsed light source can be stopped if a preselected skin temperature is reached. This measurement is relatively easy since the temperature threshold for pulsed heating that may cause skin scarring is on the order of 50°C or more, which is easily measurable using infra-red emission.

The depth of heat penetration depends on the light absorption and scattering in the different layers of the skin and the thermal properties of the skin. Another important parameter is pulse-width. For a pulsed light source, the energy of which is absorbed in an infinitesimally thin layer, the depth of heat penetration (d) by thermal conductivity during the pulse can be written as shown in Equation 1:

$$(\text{Eq.1}) \qquad d = 4 \, [k\Delta t/C\rho]^{1/2}$$

where

k = heat conductivity of the material being illuminat-ed;
$\Delta t$ = the pulse-width of the light pulse;
C = the heat capacity of the material;

$\rho$= density of the material.

It is clear from Equation 1 that the depth of heat penetration can be controlled by the pulse-width of the light source.

Thus, a variation of pulse-width in the range of $10^{-5}$ sec to $10^{-1}$ sec will result in a variation in the thermal penetration by a factor of 100.

Accordingly, the flashlamp 14 provides a pulse width of from $10^{-5}$ sec to $10^{-1}$ sec.

For treatment of external skin disorders in which evaporation of the skin is the objective, a very short pulse-width is used to provide for very shallow thermal penetration of the skin. For example, a $10^{-5}$ sec pulse will penetrate (by thermal conductivity) a depth of the order of only 5 microns into the skin. Thus, only a thin layer of skin is heated, and a very high, instantaneous temperature is obtained so that the external mark on the skin is evaporated.

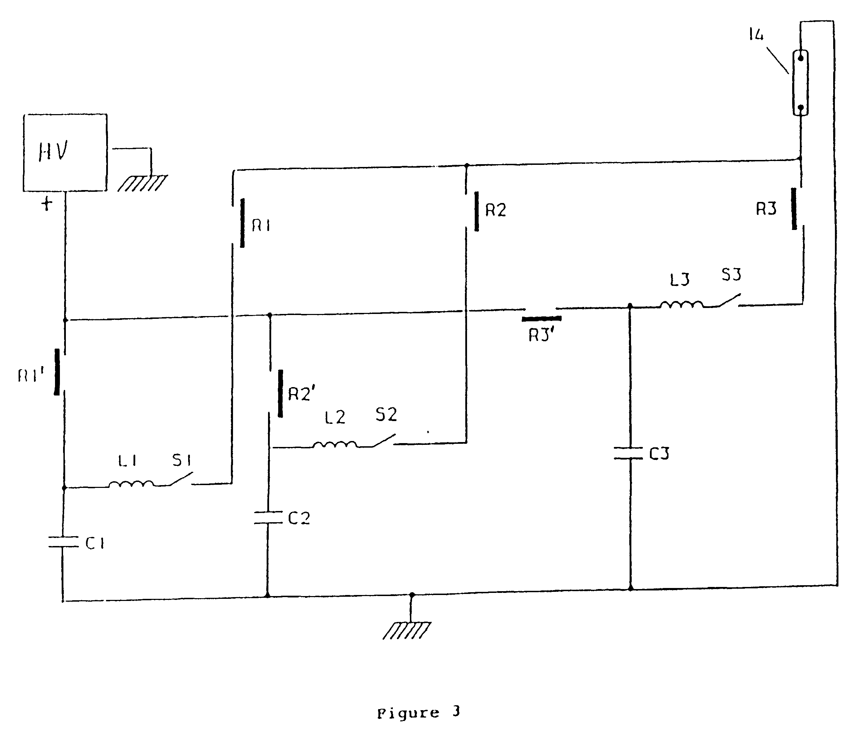

Figure 3 shows a variable pulse-width pulse forming circuit comprised of a plurality of individual pulse forming networks (PFN's) that create the variation in pulse-widths of flashlamp 14. The light pulse full width at half maximum (FWHM) of a flashlamp driven by a single element PFN with capacitance C and inductance L is approximately equal to:

$$(\text{Eq.2}) \qquad \Delta t \approx 2[LC]^{1/2} \qquad (\text{Eq.2})$$

Flashlamp 14 may be driven by three different PFN's, as shown in Figure 3. The relay contacts R1', R2' and R3' are used to select among three capacitors C1, C2 and C3 that are charged by the high voltage power supply. Relays R1, R2 and R3 are used to select the PFN that will be connected to flashlamp 14. The high voltage switches S1, S2 and S3 are used to discharge the energy stored in the capacitor of the PFN into flashlamp 14. In one embodiment L1, L2 and L3 have values of 100mH, 1mH and 5mH, respectively, and C1, C2 and C3 have values of 100mF, 1mF and 10mF, respectively.

In addition to the possibility of firing each PFN separately, which generates the basic variability in pulse-width, additional variation can be achieved by firing PFN's sequentially. If, for example, two PFN's having pulse-width $\Delta t1$ and $\Delta t2$ are fired, so that the second PFN is fired after the first pulse has decayed to half of its amplitude, then an effective light pulse-width of this operation of the system will be given by the relation: $\Delta t \approx \Delta t1 + \Delta t2$.

The charging power supply typically has a voltage range of 500V to 5kV. The relays should therefore be high voltage relays that can isolate these voltages reliably. The switches S are capable of carrying the current of flashlamp 14 and to isolate the reverse high voltage generated if the PFNs are sequentially fired. Solid-state switches, vacuum switches or gas switches can be used for this purpose.

A simmer power supply (not shown in Figure 3) may be used to keep the flashlamp in a low current conducting mode. Other configurations can be used to achieve pulse-width variation, such as the use of a single PFN and a crowbar switch, or use of a switch with closing and opening capabilities.

For external disorders a typical pulse-width of 5 microsecond is used. A 20J/cm electrical energy density input into a 5mm bore flashlamp results in an energy density on the skin of 10J/cm$^2$. Cutting off the hard UV portion of the spectrum results in 90% energy transmission, or skin exposure to an energy density of close to 10 J/cm$^2$. This energy density is high enough to evaporate external marks on the skin.

Device 10 can be provided as two units: a lightweight unit held by a physician using handle 13, with the hand-held unit containing flashlamp 14, filters 18 and iris 20 that together control the spectrum and the size of the exposed area and the detectors that measure the reflectivity and the instantaneous skin temperature. The power supply, the PFN's and the electrical controls are contained in a separate box (not shown) that is connected to the hand-held unit via a flexible cable. This enables ease of operation and easy access to the areas of the skin that need to be treated.

**Claims**

1. A therapeutic treatment device wherein an incoherent light source (14) is operable to provide a pulsed light output for treatment, said light source comprising means for providing pulses having a width in the range of between substantially 0.5 and 10 microsec and an energy density of the light on the skin of up to about 10 J/cm$^2$, whereby the light treats external disorders of the skin, such as: tattoos, pigmented lesions or birth and age marks, and wherein the therapeutic treatment device comprises a detector (22) which monitors the light reflected from the skin.

2. A treatment device as claimed in claim 1 further **characterized in that** a variable pulse width pulse forming circuit is electrically connected to said light source.

3. A treatment device as claimed in any one of the preceding claims, further **characterized in that** said light source is a flashlamp (14).

4. A treatment device as claimed in any one of the preceding claims, further **characterized in that** said light source (14) is mounted in a housing (12) suitable for being disposed adjacent a skin treatment area, said housing having a reflector (16) mounted therein proximate said light source, and said housing having an opening, with an iris (20) mounted about said opening, and at least one optical filter (18) mounted proximate said opening.

5. A treatment device as claimed in claim 4, further **characterized in that** a means (18) for providing controlled energy density, filtered, pulsed light output through said opening and said iris to a skin area for treatment is provided.

6. A treatment device as claimed in claim 4 or 5, further **characterized in that** a power supply is connected to and external of said housing, wherein said housing includes a handle (13).

## Patentansprüche

1. Eine therapeutische Behandlungsvorrichtung, in welcher eine inkohärente Lichtquelle (14) betreibbar ist, um einen gepulsten Lichtausgang fiir die Behandlung zu schaffen, wobei die Lichtquelle eine Einrichtung aufweist zum Erzeugen von Impulsen mit einer Breite im Bereich von zwischen im Wesentlichen 0,5 und 10 Mikrosekunden und einer Energiedichte des Lichtes auf der Haut von bis zu ungefähr 10 J/cm$^2$, wobei das Licht äußere Störungen der Haut wie Tätowierungen, pigmentierte Verletzungen oder Geburts- und Altersmale behandelt, und
   in welcher die therapeutische Behandlungsvorrichtung einen Detektor (22) aufweist, welcher das von der Haut reflektierte Licht überwacht.

2. Behandlungsvorrichtung nach Anspruch 1, weiter **dadurch gekennzeichnet, dass** eine Schaltung zum Bilden eines Impulses mit variabler Impulsbreite elektrisch mit der Lichtquelle verbunden ist.

3. Behandlungsvorrichtung nach einem der vorhergehenden Ansprüche, weiter **dadurch gekennzeichnet, dass** die Lichtquelle ein Blitzlicht (14) ist.

4. Behandlungsvorrichtung nach einem der vorhergehenden Ansprüche, weiter **dadurch gekennzeichnet, dass** die Lichtquelle (14) in einem Gehäuse (12) montiert ist, welches in der Nähe eines Hautbehandlungsbereiches angeordnet werden kann, wobei in dem Gehäuse nahe der Lichtquelle ein Reflektor (16) montiert ist und das Gehäuse eine Öffnung aufweist mit einer über der Öffnung montierten Iris (20) und mindestens einem nahe zu der Öffnung montierten optischen Filter (18).

5. Behandlungsvorrichtung nach Anspruch 4, weiter **dadurch gekennzeichnet, dass** eine Einrichtung (18) vorgesehen ist zum Erzeugen einer gesteuerten Energiedichte, eines gefilterten, gepulsten Lichtausganges durch die Öffnung und die Iris zu einem Hautbehandlungsbereich.

6. Behandlungsvorrichtung nach Anspruch 4 oder 5, weiter **dadurch gekennzeichnet, dass** eine Spannungsquelle mit dem Gehäuse verbunden und außerhalb des Gehäuses angeordnet ist, wobei das Gehäuse einen Handgriff (13) aufweist.

## Revendications

1. Un appareil de traitement thérapeutique dans lequel
   une source de lumière non cohérente (14) est activable pour délivrer une sortie lumineuse pour traitement, ladite source lumineuse comprenant des moyens pour délivrer des impulsions ayant une largeur dans la plage entre substantiellement 0,5 et 10 microsecondes et une densité d'énergie de la lumière sur la peau allant jusqu'à environ 10 J/cm$^2$, de sorte que la lumière traite les troubles externes de la peau tels que : tatouages, lésions pigmentées ou marques congénitales ou de vieillesse, et
   dans lequel l'appareil de traitement thérapeutique comprend un détecteur (22) qui surveille la lumière réfléchie par la peau.

2. Un appareil de traitement tel que revendiqué dans la revendication 1 **caractérisé en outre en ce qu'**un circuit de formation d'impulsions de largeur d'impulsion variable est électriquement relié à ladite source lumineuse.

3. Un appareil de traitement tel que revendiqué dans l'une quelconque des revendications précédentes, **caractérisé en outre en ce que** ladite source lumineuse est une lampe-éclair (14).

4. Un appareil de traitement tel que revendiqué dans l'une quelconque des revendications précédentes, **caractérisé en outre en ce que** ladite source lumineuse (14) est montée dans un boîtier (12) apte à être disposé adjacent à une zone de traitement de la peau, ledit boîtier ayant un réflecteur (16) qui y est monté à proximité de ladite source lumineuse, et ledit boîtier possédant une ouverture, avec un iris (20) monté autour de ladite ouverture, et au moins un filtre optique (18) monté à proximité de ladite ouverture.

5. Un appareil de traitement tel que revendiqué dans la revendication 4 **caractérisé en outre en ce qu'**il est prévu un moyen (18) pour délivrer une sortie lumineuse impulsionnelle filtrée de densité d'énergie contrôlée au travers de ladite ouverture et dudit iris vers une zone de la peau pour traitement.

**6.** Un appareil de traitement tel que revendiqué dans la revendication 4 ou 5 **caractérisé en outre en ce qu'**une alimentation de puissance est reliée au, et à l'extérieur du, dit boîtier, dans lequel ledit boîtier comprend une poignée (13).

Figure 1

Figure 2

Figure 3